# EUROPEAN PATENT SPECIFICATION

(11) **EP 1 483 371 B1**
(45) Date of publication and mention of the grant of the patent: **27.06.2007**
(21) Application number: 03705473.1
(22) Date of filing: 19.02.2003
(51) Int. Cl.: C12N 5/08

(54) **ISOLATION AND CULTURE-EXPANSION METHODS OF MESENCHYMAL STEM/PROGENITOR CELLS FROM UMBILICAL CORD BLOOD, AND DIFFERENTIATION METHOD OF UMBILICAL CORD BLOOD-DERIVED MESENCHYMAL STEM/PROGENITOR CELLS INTO VARIOUS MESENCHYMAL TISSUES**
VERFAHREN ZUR ISOLIERUNG UND KULTUREXPANSION MESENCHYMALER STAMM-/VORLÄUFERZELLEN AUS NABELSCHNURBLUT SOWIE VERFAHREN ZUR DIFFERENZIERUNG VON AUS NABELSCHNURBLUT STAMMENDEN MESENCHYMALEN STAMM-/VORLÄUFERZELLEN IN UNTERSCHIEDLICHE MESENCHYMGEWEBE
ISOLATION ET DE CULTURE-EXPANSION DE CELLULES SOUCHES OU PROGENITRICES MESENCHYMATEUSES TIREES DU SANG DU CORDON OMBILICAL, ET DIFFERENTIATION DE CES CELLULES DANS DIVERS TISSUS MESENCHYMATEUX

(30) Priority: 19.02.2002 KR 2002008639
(43) Date of publication of application: 08.12.2004
(73) Proprietor: Medipost, Co., Ltd., Seocho-gu 137-070 Seoul (KR)
(72) Inventor: HA, Chul-Won, 135-785 Seoul (KR); YANG, Yoon-Sun, 138-798 Seoul (KR); YANG, Sung-Eun, 204-1104, Yoocheon Fine Apt., Seongnam-si, 463-720 Kyungki-do (KR)
(74) Representative: Pohlman, Sandra M.
(86) International application number: PCT/KR2003/000339
(87) International publication number: WO 2003/070922

(56) References cited:
- EP-A1- 1 099 754
- WO-A-02/36751
- WO-A-97/39104
- WO-A1-02/00849
- KR-A- 2001 043 797
- ERICES ALEJANDRO ET AL: "Mesenchymal progenitor cells in human umbilical cord blood" BRITISH JOURNAL OF HAEMATOLOGY, OXFORD, GB, vol. 109, no. 1, April 2000 (2000-04), pages 235-242, XP002205332 ISSN: 0007-1048
- CAMPAGNOLI CESARE ET AL: "Identification of mesenchymal stem/progenitor cells in human first-trimester fetal blood, liver, and bone marrow" BLOOD, vol. 98, no. 8, 15 October 2001 (2001-10-15), pages 2396-2402, XP002328674 ISSN: 0006-4971
- JAISWAL N ET AL: "OSTEOGENIC DIFFERENTIATION OF PURIFIED, CULTURE-EXPANDED HUMAN MESENCHYMAL STEM CELLS IN VITRO" JOURNAL OF CELLULAR BIOCHEMISTRY. SUPPLEMENT, A.R. LISS, NEW YORK, NY, US, vol. 64, no. 2, 1997, pages 295-312, XP000929561 ISSN: 0733-1959
- MACKAY A M ET AL: "CHONDROGENIC DIFFERENTIATION OF CULTURED HUMAN MESENCHYMAL STEM CELL FROM MARROW" TISSUE ENGINEERING, LARCHMONT, NY, US, vol. 4, no. 4, November 1998 (1998-11), pages 415-428, XP002942310 ISSN: 1076-3279

## Description

### Background of Invention

### Technical Field

The present invention relates to a method for the isolation and cultivation of mesenchymal stem/progenitor cells from umbilical cord blood, and a method for the differentiation of the umbilical cord blood-derived mesenchymal stem/progenitor cells into various mesenchymal tissues.

### Background Art

As the treatment methods for damaged tissues and organs by chronic disease and cancer, there are mainly two therapeutical options such as drug medication and surgical operation. However, these techniques have problems in that they are mostly only symptomatic treatment of mitigating only symptoms, often cause surgical complications, and impose a significant economic burden upon long-term treatment.

Recently, as one expedient for treating the damaged tissues and organs, which overcomes the prior problems and can show more excellent treatment effect, there is a new attention for a method which uses the cells capable of being self-renewing and differentiating as a source of transplantation for the damaged tissues and organs.

Typical examples of such cells include mesenchymal stem cells, progenitor cells, and hemopoietic stem cells. The hemopoietic stem cells are differentiated into intravascular blood cells including red blood cells, leucocytes, and platelets, whereas the mesenchymal stem/progenitor cells are multipotent stem cells that can be differentiated into various cells.

The mesenchymal stem/progenitor cells can be differentiated into various cells and tissues constituting the human body, including marrow stromal cells, chondrocytes, osteoblasts, adipocytes, myocytes, tenocytes, ligament cells, and nervous cells. Therefore, they are highlighted as the most important cells in view of the practical use of regenerative medicine.

So far, bone marrow is the main source of the mesenchymal stem/progenitor cells.

Bone morrow is rich in mesenchymal stem/progenitor cells, but collection of bone marrow is an invasive technique including pricking with a biopsy needle several times and thus has a difficulty in practical use. Furthermore, the bone marrow collection requires general anesthesia upon a surgical operation so that it give a patient significant mental and physical burdens and also significant pain upon a surgical operation. Because of the difficulties in this collection process, the construction of infrastructure including a bone marrow storage bank is impracticable.

On the other hand, the collection of umbilical cord blood can be simply conducted after childbirth and does not cause any injury to mother and baby and thus has a high possibility of its practical use. Moreover, storage and banking of the umbilical cord blood becomes so general and active progress to the public that it is easy to seek its donor.

Umbilical cord blood is a good source of hemopoietic stem cells, and the transplantation of the hemopoietic stem cells using the umbilical cord blood is clinically activated. However, since whether the umbilical cord blood can be a good source of the mesenchymal stem/progenitor cells is not yet established, the present invention aims to provide a technique for the isolation and cultivation of the mesenchymal stem/progenitor cells from the umbilical cord blood and to demonstrate the characteristics of the umbilical cord blood-derived cells as the mesenchymal stem/progenitor cells.

The techniques of obtaining the mesenchymal stem/progenitor cells from the umbilical cord blood must be considered that it is capable of isolating and culturing only the mesenchymal stem/progenitor cells while maintaining high purity and excellent viability of the cells, since various cells including hematopoietic blood cells are present in the umbilical cord cells, and the mesenchymal stem/progenitor cells are only a very small portion thereof.

As the technique for the isolation and cultivation of the mesenchymal stem/progenitor cells, Ficoll-Hypaque centrifugation is mainly used. However, this technique has a problem in that it allows only leucocytes among various cells present in the umbilical cord blood to be removed so that substantially available mesenchymal stem/progenitor cells among the isolated cells are very small in their number and also influenced by other cells during subcultivation, thereby reducing their viability.

Owing to this reduction in number and quality of these cells, the induction of differentiation of these cells into mesenchymal tissues is not well accomplished, and conditions for the differentiation of these cells into certain tissues are not established.

Accordingly, there are needs for a method for the efficient isolation and cultivation of the mesenchymal stem/progenitor cells from the umbilical cord blood, and a method for the differentiation of these cells into the mesenchymal tissues.

The present invention aims to provide a method for efficiently isolating and cultivating the mesenchymal stem/progenitor cells from the umbilical cord blood through antigen-antibody reaction using mesenchymal stem/progenitor cell-related antibodies, and also to provide a method for the differentiation of these cells into the mesenchymal tissues.

### Disclosure of Invention

The present invention provides a method for the isolation and cultivation of the mesenchymal stem/progenitor cells from the umbilical cord blood.

The method of cell isolation and cultivation according to the present invention comprises the steps of: overlaying umbilical cord blood onto Ficoll-Hypaque solution; centrifuging the umbilical cord blood on the Ficoll-Hypaque solution to obtain mononuclear cells; reacting cells obtained by monolayer culture of the mononuclear cells with antibodies to mesenchymal stem/progenitor cell-related antigens for a predetermined period of incubation time; isolating only cells bound to their corresponding antibodies using a cell sorter; and cultivating the isolated cells.

In the method for isolating and cultivating the mesenchymal stem/progenitor cells according to the present invention, the umbilical cord blood is defined as the blood collected from umbilical vein connecting the placenta to a fetus in mammals. In the method of cell isolation and cultivation according to the present invention, human umbilical cord blood is preferably used.

The Ficoll-Hypaque solution that is used in the method of cell isolation and cultivation according to the present invention preferably has a density of 1.077 g/ml.

The antibodies to the mesenchymal stem/progenitor cell-related antigens, which are used in the method of cell isolation and cultivation of the present invention, are one or more selected from antibodies for cell surface antigens that are expressed from the mesenchymal stem/progenitor cells. Specifically, they are one or more selected from antibodies for CD105, stro-1, SH3 and SH4, and such antibodies are preferably used all together in order to increase purity to the maximum.

The antibodies to the mesenchymal stem/progenitor cell-related antigens, which are used in the method of cell isolation and cultivation of the present invention, have a suitable marker attached thereto, which varies according to the characteristic of the cell sorter. Specifically, when a magnetic cell sorter is used, antibodies having a magnetic microbead attached thereto are used. When a FACsorter is used, there are used antibodies to which a fluorochrome, such as fluorescein isothiocyanate (FITC), phycoerythrin (PE), PerCP or the like, is attached.

In the cells isolated as described above, there will be present only the cells expressing the antigens, i.e., the mesenchymal stem/progenitor cells.

In the present invention, the immune response between antigens and antibodies is used through the use of the antibodies to the mesenchymal stem/progenitor cell-specific antigens as described above, so that only the mesenchymal stem/progenitor cells among various cells present in the umbilical cord blood are isolated and cultivated. Thus, the substantial number of available stem cells is increased.

The present invention provides umbilical cord blood-derived mesenchymal stem/progenitor cells obtained by the method of cell isolation and cultivation as described above.

In the present invention, the progenitor cells are defined as all progenitor cells which can be obtained during the differentiation of the umbilical cord blood-derived mesenchymal stem/progenitor cells into chondrocytes and osteoblasts.

The umbilical cord blood-derived mesenchymal stem/progenitor cells have immunophenotypic characteristics in that they show a positive response to antibodies for CD29, CD49e, CD44, CD54, CD 13, CD90, SH2, SH3 and SH4 antigens, and show a negative response to antibodies for CD45, CD34, CD 14, HLA-DR, CD31, CD51/61, CD49d, CD106, and CD64 antigens.

Hereinafter, the immunophenotypic characteristic of the umbilical cord blood-derived mesenchymal stem/progenitor cells according to the present invention will be described in detail.

The umbilical cord blood-derived mesenchymal stem/progenitor cells of the present invention show negative response to CD45, CD34 and CD14 as hematopoietic antigens and HLA-DR as a histocompatibility antigen so that they can minimize the rejection response that is the greatest problem in tissue or organ transplantation. Thus, the umbilical cord blood-derived mesenchymal stem/progenitor cells of the present invention are useful as a source of cells for allogeneic transplantation and also as universal donor cells.

The umbilical cord blood-derived mesenchymal stem/progenitor cells of the present invention show a negative response to CD31 as an endothelial cell-associated antigen, and for CD51/61 as an osteoclast-associated antigen.

Thus, when the umbilical cord blood-derived mesenchymal stem/progenitor cells of the present invention is used in tissue transplantation, side effects are minimized that can be caused by the production of undesired blood vessels or the differentiation of cells into osteoclasts during the production of chondrocytes and osteoblasts.

The umbilical cord blood-derived mesenchymal stem/progenitor cells of the present invention show a positive response to CD 29 and CD49e as integrin receptor-associated antigens, and a negative response to CD49d.

The umbilical cord blood-derived mesenchymal stem/progenitor cells of the present invention show a positive response to CD44 and CD54 as matrix receptor-associated antigens, and a negative response to CD106.

The umbilical cord blood-derived mesenchymal stem/progenitor cells of the present invention show a positive response to antibodies for other antigens, namely CD 13 and CD90, and a negative response to an antibody for a CD 64 antigen.

The umbilical cord blood-derived mesenchymal stem/progenitor cells of the present invention show a positive response to antibodies for SH2, SH3 and SH4 as mesenchymal stem/progenitor cell-associated antigens, and this immunophenotypic characteristic is stably maintained even after several passages.

The immunophenotypic characteristics of the inventive cells as described above are identical to the immunophenotypic characteristic of typical mesenchymal stem/progenitor cells.

The umbilical cord blood-derived mesenchymal stem/progenitor cells of the present invention have self-renewal capability under a suitable condition so that they can continue to expand while they are not differentiated into certain cells or tissues.

The umbilical cord blood-derived mesenchymal stem/progenitor cells of the present invention are cells originated from a population younger than cells originated from mesenchymal stem cells isolated from general mesenchymal tissues including marrow, muscle and skin tissues, so that they have more excellent differentiation capability.

Owing to this multipotency, the cells of the present invention can be differentiated into mesenchymal tissues, such as osteoblasts, chondrocytes, adipocytes, myocites, tenocytes and so on, under suitable conditions.

Specifically, the present invention provides a method for the differentiation of the umbilical cord blood-derived mesenchymal stem/progenitor cells into mesenchymal cells.

The method of cell differentiation according to the present invention comprises cultivating the umbilical cord blood-derived mesenchymal stem/progenitor cells in cell differentiation medium for predetermined periods of time under suitable conditions, which vary depending to the kind of the mesenchymal tissue cells to be differentiated.

In the method of cell differentiation according to the present invention, the mesenchymal cells can be specifically chondrocytes or osteoblasts. Compositions of chondrogenic differentiation medium and osteogenic differentiation medium that are used in the present invention are given in Tables 1 and 2 below, respectively.

**Table 1: Composition of chondrogenic differentiation medium according to the present invention**

| Components | | Concentration |
|---|---|---|
| TGF-β III | | 10 ng/mℓ |
| | Bovine insulin | 6.25 µg/mℓ |
| ITS-Plus | Transferrin | 6.25 µg/mℓ. |
| | Selenous acid | 5.35 µg/mℓ |
| | Linoleic acid | 1.25 µg/mℓ |
| | Bovine serum albumin (BSA) | 100 µg/mℓ |
| Sodium pyruvate | | 100 nM |
| Dexamethasone | | 100 nM |
| Ascorbic acid 2-phosphate | | 50 µg/mℓ |
| Proline | | 40 µg/mℓ |

**Table 2: Composition of osteogenic differentiation medium according to the present invention**

| Components | Concentration |
|---|---|
| Dexamethasone | 0.1 µM |
| β -glycerol phosphate | 10 mM |
| Ascorbic acid 2-phosphate | 50 µM |

The components indicated in Tables 1 and 2 are used after added to one selected from conventional cell culture mediums, including DMEM, α-MEN, McCoys 5A medium, Eagle's basal medium, CMRL medium, Glasgow minimal essential medium, Ham's F-12 medium, Iscove's modified Dulbecco's medium, Liebovitz' L-15 medium, RPMI 1640 medium and so on. In the case of the chondrogenic differentiation medium, DMEM is preferably used, and in the case of the osteogenic differentiation medium, α-MEM is preferably used.

Furthermore, in addition to the components as described above, the cell differentiation medium that is used in the present invention may additionally contain one or more assistants, if necessary. Such assistants include a growth factor, and horse or human serum, and also antibiotics and antifungal agents, including penicillin G, streptomycin sulfate, amphotericin B, gentamycin and nystatin, which can be added to prevent microorganism contamination.

As evident from Examples below, according to the method of cell isolation and cultivation of the present invention, the mesenchymal stem/progenitor cells with high purity and excellent viability can be obtained from the umbilical cord blood.

The mesenchymal stem/progenitor cells of the present invention can be differentiated into various mesenchymal tissues so that they express type II collagen, type X collagen and aggrecan genes, as typical markers of the chondrocytes, under suitable chondrogenic medium and conditions.

Moreover, the umbilical cord blood-derived mesenchymal stem/progenitor cells of the present invention can express osteocalcin, osteopontin and alkaline phosphatase genes, as typical markers of the osteblasts, under suitable osteogenic medium and conditions, and allows extracellular accumulation of calcium in the same manner as the osteoblasts.

As a result, the method of cell isolation and cultivation of the present invention can be used for the mass production of the mesenchymal stem/progenitor cells that are so-called multipotent cells. Also, this will exhibit its utility in accordance with the expansion of an umbilical cord blood storage system, which is currently actively conducted by an umbilical cord blood storage bank.

Furthermore, the umbilical cord blood-derived mesenchymal stem/progenitor cells obtained by the method of cell isolation and cultivation of the present invention can be differentiated into various tissues, if necessary. Thus, they can bring an important development in the treatment of injured mesenchymal tissues whose renewal was difficult.

### Brief Description of the Drawings

FIG. 1 is a drawing showing a collection bag containing umbilical cord blood collected from umbilical vein;
FIG. 2 is a drawing showing a morphological characteristics of umbilical cord blood-derived mesenchymal stem/progenitor cells according to the present invention;
FIG. 3 is a drawing showing the result of a test to examine if umbilical cord blood-derived mesenchymal stem/progenitor cells of the present invention express hematopoietic antigens and histocompatibility antigens;
FIG. 4 is a drawing showing the result of a test to examine if umbilical cord blood-derived mesenchymal stem/progenitor cells of the present invention express endothelial cell-associated antigens and osteoclast-associated antigens;
FIG. 5 is a drawing showing the result of a test to examine if umbilical cord blood-derived mesenchymal stem/progenitor cells of the present invention express integrin receptor-associated antigens;
FIG. 6 is a drawing showing the result of a test to examine if umbilical cord blood-derived mesenchymal stem/progenitor cells of the present invention express matrix receptor-associated antigens;
FIG. 7 is a drawing showing the result of a test to examine if umbilical cord blood-derived mesenchymal stem/progenitor cells of the present invention express other antigens;
FIG. 8 is a drawing showing the result of a test to examine if surface antigens of mesenchymal stem/progenitor cells are expressed in umbilical cord blood-derived mesenchymal stem/progenitor cells of the present invention;
FIG. 9 is a drawing showing the result of immunostaining for cartilage-associated proteins after umbilical cord blood-derived mesenchymal stem/progenitor cells of the present invention were differentiated into chondrocytes;
FIG. 10 is a drawing showing the result of RT-PCR for cartilage-associated genes after umbilical cord blood-derived mesenchymal stem/progenitor cells of the present invention were differentiated into chondrocytes;
FIG. 11 is a drawing showing the result of histochemical staining for bone-associated proteins and inorganic substances after umbilical cord blood-derived mesenchymal stem/progenitor cells of the present invention were differentiated into osteoblasts; and
FIG. 12 is a drawing showing the result of RT-PCR for bone-associated genes after umbilical cord blood-derived mesenchymal stem/progenitor cells of the present invention were differentiated into osteoblasts.

### Best Mode for Carrying Out the Invention

The present invention will hereinafter be described in further detail by examples. It should be borne in mind that the present invention is not limited to or by the examples.

### Example 1: Isolation and cultivation of mesenchymal stem/progenitor cells from umbilical cord blood according to the present invention

### 1) Isolation and ex vivo cultivation of mesenchymal stem/progenitor cells from umbilical cord blood

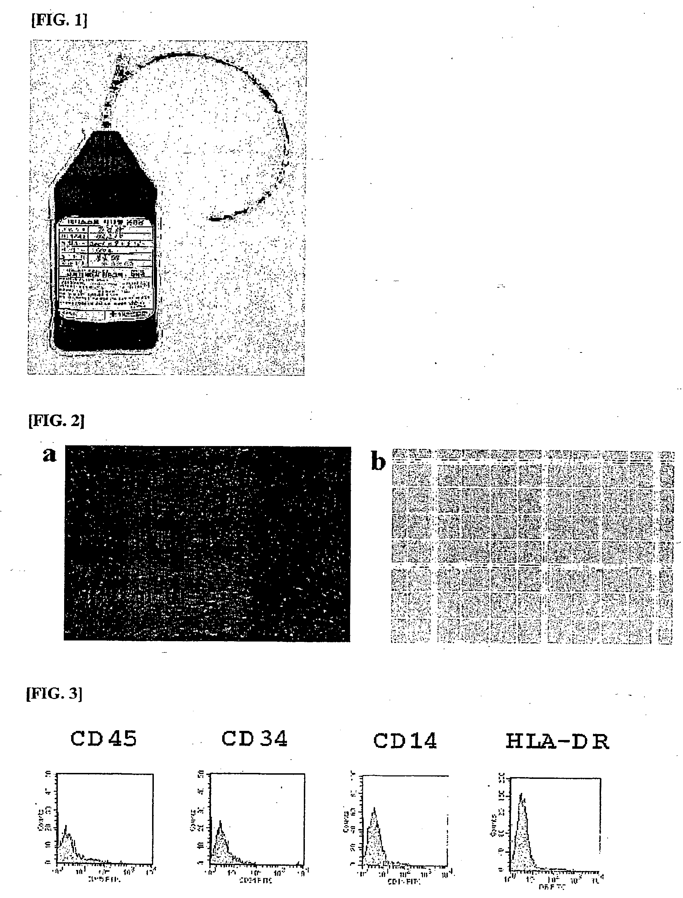

Umbilical cord blood was collected from umbilical vein after childbirth. In collecting the umbilical cord blood, after the umbilical cord was sufficiently sterilized with alcohol and betadine, the umbilical vein was pricked with a 16G needle connected to an umbilical cord blood-collection bag containing 23ml of a CDPA-1 anticoagulant such that the umbilical cord blood was collected into the collection bag by gravity (see, FIG. 1).

After 15ml Ficoll-Hypaque (density: 1.077 g/ml) was placed in a 50ml conical tube, 25ml umbilical cord blood collected as described above was slowly overlaid onto Ficoll-Hypaque and centrifuged at 400xg for 40 minutes at room temperature to form a mononuclear cell layer. After removing the supernatant, the mononuclear cell layer was transferred to a fresh tube. To the mononuclear cells, 30ml phosphate buffered saline (PBS) containing 2% fetal bovine serum was added, centrifuged at 200xg for 10 minutes and washed.

After the washing was repeated two times, the mononuclear cell layer was added with 30ml NH₄Cl-Tris solution, left to stand for 15 minutes, and centrifuged at 200xg for 10 minutes at room temperature. After removing the supernatant, the mononuclear cell layer was added with 30ml 2%FBS-PBS, centrifuged at 200xg for 10 minutes at room temperature and washed.

After this procedure was repeated again, the mononuclear cell layer obtained by supernatant removal was added with 10ml basal medium (a-MEM or DMEM medium) containing 10% FBS and well mixed.

The mononuclear cells isolated as described above were measured for their viability and cell count, introduced into a cell culture vessel along with basal medium at a suitable number (5 X 10⁵ to 1 X 10⁶ cells/cm²), and then incubated in 5% CO₂ incubator. Then, the cells were monolayer-cultured at 37 °C.

The appearance of a colony was observed with a microscope everyday to examine if the colony is well attached and grows in a monolayer on the bottom of the culture vessel.

After the cells reached 90% confluency, the medium was removed using a suction pump and a pipette, and then the cells were washed with PBS from which calcium and magnesium had been removed. The washed cells were added with 0.25% Trypsin/EDTA solution, and left to stand for 10 minutes at 37 °C in 5% CO₂ incubator. Cells detached from the cell culture vessel were collected in a tube again, centrifuged at 200xg for 10 minutes at room temperature and washed.

The washed cells were reacted with antibodies to mesenchymal stem/progenitor cell-specific antigens for a given period of incubation time. The antibodies to the mesenchymal stem/progenitor cell-specific antigens were antibodies to CD105, stro-1, SH3 and SH4, in which each of the antibodies has a magnetic bead or a fluorochrome attached thereto, such as fluorescein isothiocyanate (FITC), phycoerythrin (PE) or PerCP and so on.

After reaction with the antibodies, the mesenchymal stem/progenitor cells bound to their corresponding antibodies were isolated using cell separation equipment such as magnetic cell sorter or FACsorter.

The cells isolated as described above were added with 10ml basal medium containing 10% FBS, thoroughly mixed and measured for their viability and cell count. Basal medium and the mesenthymal stem/progenitor cells of a suitable number (4 to 5 x 10⁴ cells/cm²) were plated into a cell culture vessel, and cultivated at 37 °C, in 5% CO₂ incubator.

Thereafter, whenever the cells reached 100% confluency, repeated subcultivation was conducted so that the umbilical cord blood-derived mesenchmal stem/progenitor cells were expanded *ex vivo.*

FIG. 2 shows the morphological characteristics of the cells isolated by the method of the present invention. In FIG. 2a, the cells isolated by the inventive method grew in the form of a spindle shape, as a typical shape of the mesenchymal stem/progenitor cells, and a homogeneous fibroblast-like colony shape. As shown in FIG. 2b, the cells of the present invention were stained with Trypan blue and measured for their viability, and results showed a very excellent viability of 98-99%.

As a result, the method of the present invention allows efficient isolation and cultivation of the mesenchymal stem/progenitor cells from the umbilical cord blood.

### 2) Analysis of characteristics of umbilical cord blood-derived mesenchymal stem/progenitor cells obtained by the present invention

In order to examine if the umbilical cord blood-derived cells obtained by the present invention have the characteristic of the mesenchymal stem/progenitor cells, the expression pattern of cell surface antigens of the cells obtained in 1) of Example 1 were analyzed as follows.

Antigens whose immunophenotypes are examined in this Example were CD45, CD34 and CD14 as hematopoietic antigens, HLA-DR as a histocompatibility antigen, CD31 as an endothelial cell-associated antigen, CD51/61 as an osteoclast-associated antigen, CD29, CD49d and CD49e as integrin receptor-associated antigens, CD44, CD54 and CD106 as matrix receptor-associated antigens, SH2, SH3 and SH4 as mesenchymal stem/progenitor cell-specific antigens, and CD 13, CD64 and CD90 as other antigens.

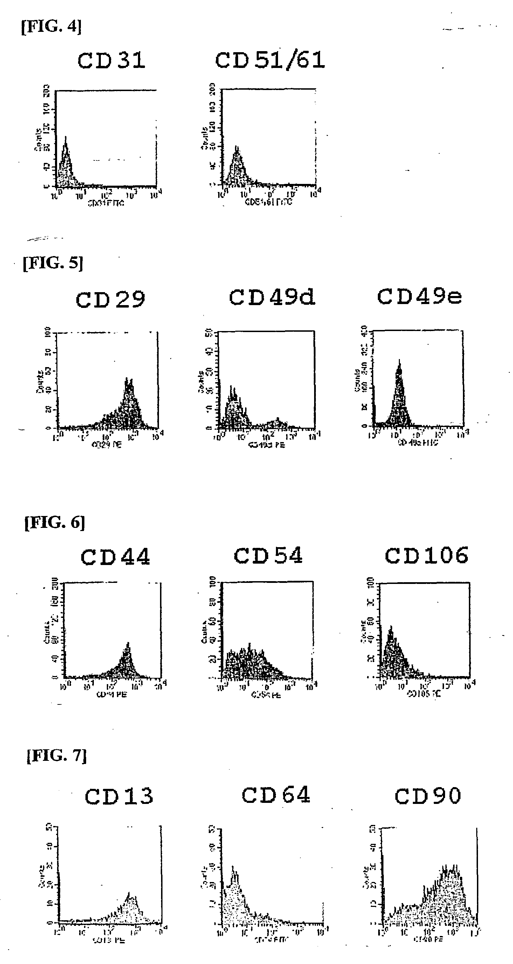

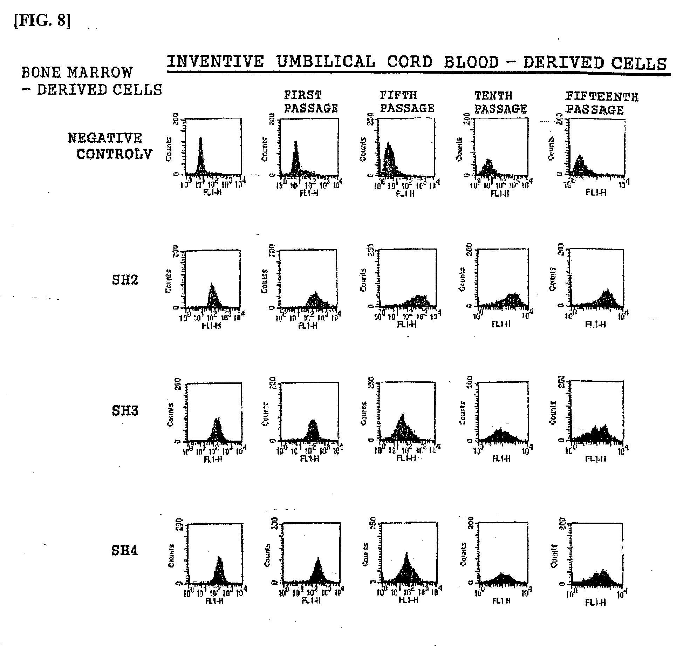

2 x 10⁶ cells cultivated in the above 1) were washed with PBS solution containing 2% FBS, and reacted with antibodies corresponding to the respective antigens at room temperature. The expression of the antigens was examined using a flow cytometer, and the results thereof are shown in FIGS. 3 to 7. Namely, FIG. 3 shows results for the hematopoietic antigens and the histocompatibility antigens, FIG. 4 shows results for the endothelial cell-associated antigens and osteoclasts-associated antigens, FIG. 5 shows results for the integrin receptor-associated antigens, FIG. 6 shows results for the matrix receptor-associated antigens, and FIG. 7 shows results for the other antigens.

As shown in FIG. 3, the umbilical cord blood-derived mesenchymal stem/progenitor cells of the present invention showed a negative response to antibodies for CD45, CD34 and CD14 as hematopoietic antigens, and HLA-DR as a histocompatibility antigen.

Thus, the umbilical cord blood-derived mesenchymal stem/progenitor cells of the present invention are deficient in the hematopoietic antigens and the histocompatibility antigen, so that they can minimize rejection that is a problem in tissue transplantation.

As shown in FIG. 4, the umbilical cord blood-derived mesenchymal stem/progenitor cells of the present invention showed a negative response to antibodies for CD31 as an endothelial cell-associated antigen, and CD51/61 as an osteoclast-associated antigen.

Thus, it can be found that, when the umbilical cord blood-derived mesenchymal stem/progenitor cells of the present invention are used in tissue transplantation, there are no side effects that can be caused by the production of undesired blood vessels and the differentiation of cells into the osteoclasts during chondrocyte or osteoblast production.

As shown in FIG. 5, the umbilical cord blood-derived mesenchymal stem/progenitor cells of the present invention showed a positive response to antibodies for CD 29 and CD49e as integrin receptor-associated antigens while showing a negative response to an antibody for CD49d antigen.

As shown in FIG. 6, the umbilical cord blood-derived mesenchymal stem/progenitor cells of the present invention showed a positive response to antibodies for CD44 and CD54 as matrix receptor-associated antigens while showing a negative response to an antibody for CD106 antigen.

As shown in FIG. 7, the umbilical cord blood-derived mesenchymal stem/progenitor cells of the present invention showed a positive response to antibodies for other antigens, i.e., CD13 and CD90, while showing a negative response to an antibody for to CD64 antigen.

Also, there was conducted a test to examine if each of the first, fifth, tenth and fifteenth passage cultures of the umbilical cord blood-derived mesenchymal stem/progenitor cells of the present invention express SH2, SH3 and SH4 as typical surface antigens of the mesenchymal stem/progenitor cells. As a result, as shown in FIG. 8, it could be found that the cells of the present invention showed a positive response to these antigens, like the first passage culture of the bone marrow-derived mesenchymal stem/progenitor cells, even after several subcultivations, and this immunophenotype was stably maintained even after several passages.

The immunophenotypes shown in FIGS. 3-8 of the umbilical cord blood-derived mesenchymal stem/progenitor cells of the present invention are identical to those of the prior mesenchymal stem/progenitor cells. This suggests that the cells isolated from the umbilical cord blood by the method of the present invention have excellent characteristics for the mensenchymal stem/progenitor cells.

### Example 2: Differentiation of umbilical cord blood-derived mesenchymal stem/progenitor cells of the present invention into chondrocytes

### 1) Differentiation of umbilical cord blood-derived mesenchymal stem/progenitor cells of the present invention into chondrocytes

In order to examine if the umbilical cord blood-derived mesenchymal stem/progenitor cells of the present invention have the characteristic of differentiating into mesenchymal tissues, the differentiation of these cells into chondrocytes was induced.

The medium used in differentiation into the chondrocytes had the composition given in Table 1 above, and the cells were differentiated in pellet cultures. The medium was replaced every three days, and cells were sampled at one-week intervals after differentiation induction, and subjected to immunomarker expression analysis and molecular biological analysis.

### 2) Immunochemical analysis of the chondrogenic differentiated tissues

After differentiation into the chondrocytes, the cells of the present invention were immunostained as follows in order to examine that they express type II collagen, as a chondrocyte-specific antigen.

Cell pellet samples collected at one-week intervals after inducing differentiation into chondrocytes were embedded in paraffin, or frozen and sectioned in order to sufficiently maintain the antigenicity of epitopes. Then, the pellet tissues were immobilized on slides to a thickness of 3-5 µm and immunostained.

The respective slides were treated with hydrogen peroxide for 5 minutes to remove peroxidase present within the cells, and then treated with a protein blocking reagent for 5 minutes.

Thereafter, they were incubated with rat monoclonal antibodies for 10 minutes. After washing, they were treated with streptavidin peroxidase for 10 minutes. Next, they were treated with chromogens to cause color reaction, and counterstained with hematoxilin.

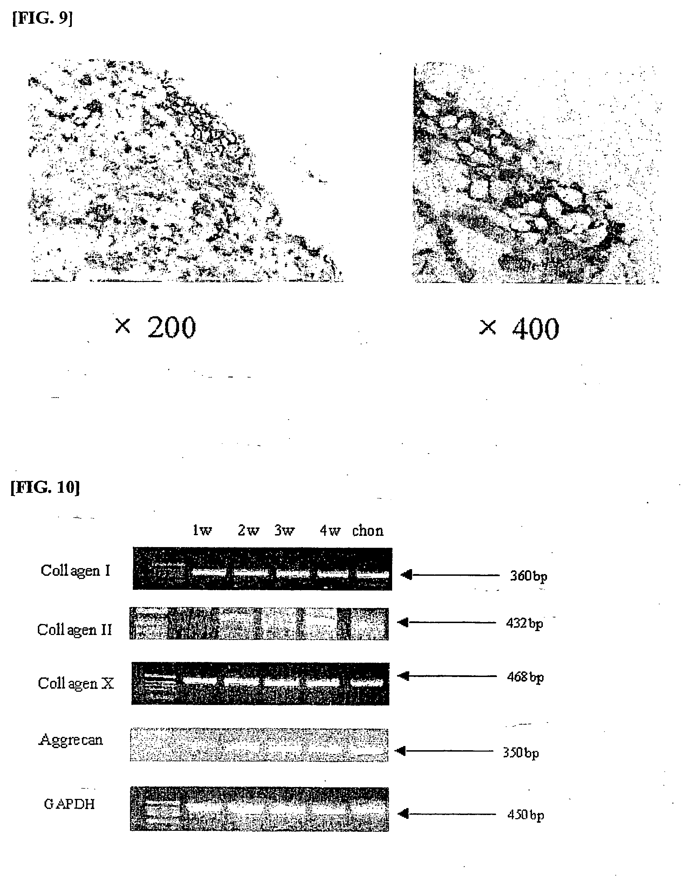

As a result, one week after inducing the differentiation of the umbilical cord blood-derived mesenchymal stem/progenitor cells of the present invention into the chondrocytes, positive findings were observed in a portion of the pellets. Also, as shown in FIG. 9, three weeks after inducting the differentiation, positive findings were observed in the whole pellets.

Considering the fact that the differentiated cells secrete the type II collagen as an important component of extracellular matrix (ECM) as described above, it is believed that the cells of the present invention can sufficiently perform functions of the chondrocytes.

### 3) Molecular biological analysis of the chondrogenic differentiated tissues

After inducing the differentiation of the inventive cells into chondrocytes, reverse transcription-polymerase chain reaction (RT-PCR) was conducted as follows in order to examine if the inventive cells express chondrocyte-specific genes.

Cell pellets collected at one-week intervals after inducing differentiation into chondrocytes were treated with Trizol R for 5 minutes and treated with chloroform, followed by centrifugation at 15000 rpm for 15 minutes. The supernatant was taken and added with isopropanol so as to precipitate RNA.

In RT reaction, RNA obtained as described above, 1µl oligo d(T) primer, 1µl dNTP mix solution, and RNase-free water were mixed and reacted for 5 minutes at 65 °C. To this mixture, 4µl RT reaction buffer, 2µl DTT and 1µl RNase inhibitor were added and reacted at 42 °C for 2 minutes. After this, reverse transcriptase was added to the mixture and reacted at 42 °C for 50 minutes. cDNA obtained as described above was inactivated at 70 °C for 15 minutes and used as a PCR template.

In PCR reaction, type I collagen, type II collagen, type X collagen and aggrecan genes were used as primers. As a positive control, a human articular chondrocyte was selected, and as a negative control, a GAPDH gene that is always expressed in cells at a constant level was selected.

To each of reaction tubes, 5µl cDNA, primer, dNTP mix solution, magnesium chloride, 10-fold PCR reaction buffer, and Taq polymerase were added, to which sterilized, triply distilled water was added so as to adjust a final reaction volume to 50µl. Then, PCR reaction was conducted in the 50µl final reaction volume. The base sequence of a primer for each of the genes, and reaction conditions are given in Table 3 below.

**Table 3:**

| Genes | Base sequence of primers | PCR composition | PCR conditions |
|---|---|---|---|
| GAPDH | 5'-ACCACAGTCCATGCCATCAC-3' (forward, SEQ ID NO: 1) | | Initial denaturation at 94°C for 2min; |
| | 5'-TCCACCACCCTGTTGCTGTA-3' (reverse, SEQ ID NO: 2) | | 35 cycles of 94°C for 30sec, 60°C for 30sec, and 72°C for 30 sec; and final extension at 72°C for 7min |
| Type I collagen | 5'-CCCCCTCCCCAGCCACAAAGA-3' (forward, SEQ ID NO: 3) | | Initial denaturation at 94°C for 2min; |
| | 5'-TCTTGGTCGGTGGTGGACTCT-3' (reverse, SEQ ID NO: 4) | | 35 cycles of 94°C for 30sec, 60°C for 30sec, and 72°C for 30 sec; and final extension at 72°C for 7min |
| | | cDNA | |
| Type II collagen | 5'-TTTCCCAGGTCAAGATGGTC-3' (forward, SEQ ID NO: 5) | template: 5µl | Initial denaturation at 94°C for 2min; |
| | 5'-CITCACCACCTGTCfCACCA-3' (reverse, SEQ ID NO: 6) | primers: 2.5µl, respectively | 35 cycles of 94°C for 30sec, 55°C for 30sec, and 72°C for 30 sec; and final extension at 72°C for 7min |
| | | PCR mix | |
| Type X collagen | 5'-CCCTITITGCfGCTAGTATCC-3' (forward, SEQ ID NO: 7) | solution: 7.7µl | Initial denaturation at 94°C for 2min; |
| | 5'-CTGTTGTCCAGGTTTTCCTGGCAC-3' | | 35 cycles of 94°C for 30sec, 57°C for 30sec, and 72°C for 30 sec; and final extension at 72°C for and final extension at 72°C for 7min |
| | (reverse, SEQ ID NO: 8) | | |
| Aggrecan | 5'-TGAGGAGGGCTGGAACAAGTACC-3' | | Initial denaturation at 94°C for 2min; |
| | (forward, SEQ ID NO: 9) | | 35 cycles of 94°C for 30sec, 60°C for 30sec, and 72°C for 30 sec; and final extension at 72°C for 7min |
| | 5'-GGAGGTGGTAATTGCAGGGAACA-3' | | |
| | (reverse, SEQ ID NO: 10) | | |

The respective PCR products were electrophoresed, and the results thereof are shown in FIG. 10.

As shown in FIG. 10, the umbilical cord blood-derived mesenchymal stem/progenitor cells of the present invention expressed all the type II collagen, type X collagen and aggrecan genes from one week after inducing their differentiation into chondrocytes.

Particularly, the expression level of each of the genes was further increased with longer differentiation time. Four weeks after differentiation induction, the expression level of each of the genes was as high as the human articular chondrocyte (referred to as "chon") that is the positive control.

On the other hand, the expression level of the GAPDH gene as the negative control was constant regardless of differentiation induction time.

Accordingly, the umbilical cord blood-derived mesenchymal stem/progenitor cells of the present invention can be differentiated into the chondrocytes under suitable conditions.

### Example 3: Differentiation of umbilical cord blood-derived mesenchymal stem/progenitor cells of the present invention into osteoblasts

### 1) Differentiation of umbilical cord blood-derived mesenchymal stem/progenitor cells of the present invention into osteoblasts

In order to examine if the mesenchymal stem/progenitor cells of the present invention have the characteristics of differentiating into the osteoblasts, the differentiation of the inventive cells into the osteoblasts was induced.

The medium used in differentiation into the osteoblasts had the composition given in Table 1 above, and the cells were differentiated in monolayer-culture. The medium was replaced every three days, and cells were sampled at one-week intervals after differentiation induction, and subjected to immunomarker expression analysis and molecular biological analysis.

### 2) Histochemical analysis of osteogenic differentiated tissues

After differentiation into the osteoblasts, the cells of the present invention were histochemically stained as follows in order to examine if they express alkaline phosphatase that is an osteoblast-specific antigen.

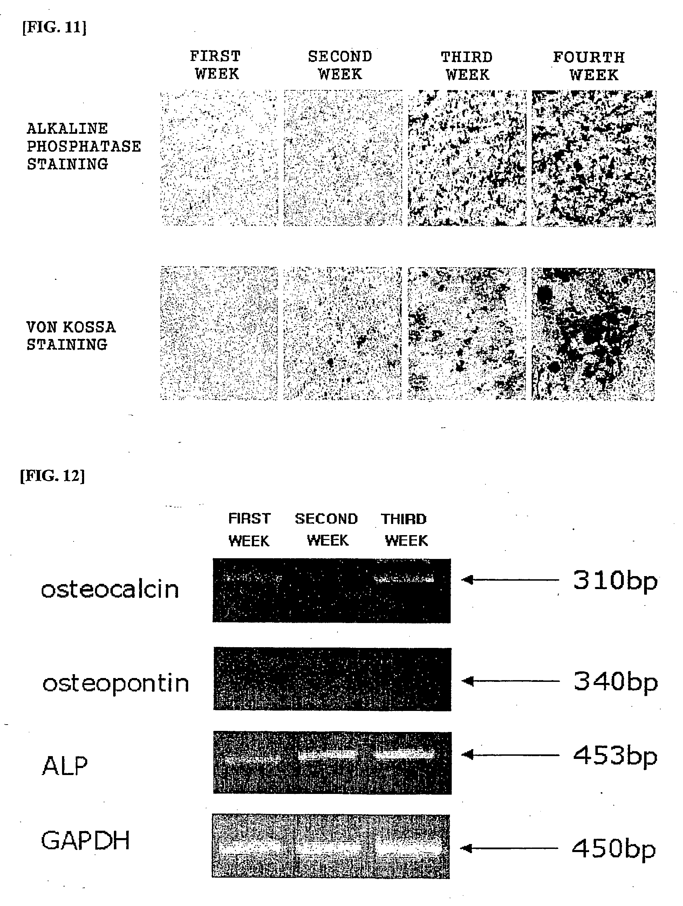

Cells, which had been collected at one-week intervals after induction of differentiation into the osteoblasts by monolayer culture, were immobilized with methanol and then histochemically stained for alkaline phosphatase as an osteoblast-specific antigen. Also, in order to determine if calcium as an extracellular component is accumulated, the cells were examined by von Kossa staining. Results are shown in FIG. 11.

As shown in FIG. 11, from one week after inducting the differentiation of the umbilical cord blood-derived mesenchymal stem/progenitor cells of the present invention into the osteoblasts, positive findings were observed in a portion of the tissues. Three to four weeks after induction of differentiation, positive findings were observed in the whole tissues.

The results of von Kossa staining showed that extracellular calcium accumulation was gradually increased.

Considering the fact that the differentiated cells express the alkaline phosphatase as an important component of the osteoblast and allow extracellular calcium accumulation as described above, it is believed that the cells of the present invention can sufficiently perform functions of the osteoblasts.

### 3) Molecular biological analysis of osteogenic differentiated tissues

After differentiation into osteoblasts, RT-PCR was conducted as follows in order to examine if the cells of the present invention express osteoblast-specific genes.

Tissues collected at one-week intervals after differentiation into osteoblasts were treated with Trizol R for 5 minutes and then treated with chloroform, followed by centrifugation at 15000 rpm for 15 minutes. The supernatant was taken and added with isopropanol so as to precipitate RNA.

In RT reaction, RNA obtained as described above, 1µl oligo d(T) primer, 1µl dNTP mix solution, and RNase-free water were mixed and reacted for 5 minutes at 65 °C. To this mixture, 4µl RT reaction buffer, 2µl DTT and 1µl RNase inhibitor were added and reacted at 42 °C for 2 minutes. After this, reverse transcriptase was added to the mixture and reacted at 42 °C for 50 minutes. cDNA thus obtained was inactivated at 70 °C for 15 minutes and used as a PCR template.

In PCR reaction, osteocalcin, osteopontin alkaline phosphatase that are osteoblast-specific genes were used as primers. As a negative control, a GAPDH gene that is always expressed in cells at a constant level was selected.

To each of reaction tubes, 5µl cDNA, primer, dNTP mix solution, magnesium chloride, 10-fold PCR reaction buffer, and Taq polymerase were added, to which sterilized, triply distilled water was added so as to adjust a final reaction volume to 50µl. Then, PCR reaction was conducted in the 50µl final reaction volume. The base sequence of a primer for each of the genes, and reaction conditions are given in Table 4 below.

**Table 4:**

| Genes | Base sequences of primers | PCR composition | PCR conditions |
|---|---|---|---|
| GAPDH | 5'-ACCACAGTCCATGCCATCAC-3' | | Initial denaturation at 94°C for 2min; 35 cycles of 94°C for 30sec, 55°C for 30sec, and 72°C for 30 sec; and final extension at 72°C for 7min |
| | (forward, SEQ ID NO: 1) | | |
| | 5'-TCCACCACCCTGTTGCTGTA-3' | | |
| | (reverse, SEQ ID NO: 2) | | |
| Osteocalcin | 5'-CATGACAGCCCTCACA-3' | cDNA template: | |
| | (forward, SEQ ID NO: 11) | 5µl | |
| | 5'-AGAGCGACACCCTAGAC-3' | | |
| | (reverse, SEQ ID NO: 12) | Primers: 2.5µl, | |
| Osteopontin | 5'-CCAAGTAAGTCCAACGAAAG-3' | respectively | |
| | (forward, SEQ ID NO: 13) | | |
| | 5'-GGTGATGTCCTCGTCTGTA-3' | PCR mix solution: | |
| | (reverse, SEQ ID NO: 14) | 7.7µl | |
| Alkaline phosphatase | 5'-TGGAGCTTCAGAGACTCAACACCA-3' | | |
| | (forward, SEQ ID NO: 15) | | |
| | 5'-ATCTCGTTGTCTGAGTACCAGTCC-3' | | |
| | (reverse, SEQ ID NO: 16) | | |

The respective PCR products were electrophoresed, and the results thereof are shown in FIG. 12.

As shown in FIG. 12, the umbilical cord blood-derived mesenchymal stem/progenitor cells of the present invention expressed all osteocalcin, osteopontin and alkaline phosphatase from one week after inducing their differentiation into the osteocytes. The expression level of each of the genes was further increased with longer differentiation time.

On the other hand, the expression level of the GAPDH gene as the negative control was constant regardless of the passage of differentiation induction time.

Accordingly, the umbilical cord blood-derived mesenchymal stem/progenitor cells of the present invention can be differentiated into the osteoblasts under suitable conditions.

### Industrial Applicability

As described above, the method of cell isolation and cultivation according to the present invention has an effect of isolating the mesenchymal stem/progenitor cells from the umbilical cord blood while maintaining high purity and viability of the cells.

The mesenchymal stem/progenitor cells, which were isolated and cultivated from the umbilical cord blood according to the present invention, can be differentiated to various mesenchymal tissues, including chondrocytes and osteoblasts, under suitable conditions.

Accordingly, the method of cell isolation and cultivation according to the present invention, and the umbilical cord blood-derived mesenchymal stem/progenitor cells isolated and cultivated thereby, are useful in the renewal and treatment of injured mesenchymal tissues.

## Claims

1. A method for the isolation, cultivation and chondrogenesis of mesenchymal stem/progenitor cells from umbilical cord blood, which comprises the steps of: overlaying umbilical cord blood onto Ficoll-Hypaque solution;
centrifuging the umbilical cord blood on the Ficoll-Hypaque solution to obtain mononuclear cells;
reacting cells obtained by monolayer culture of the mononuclear cells with antibodies to mesenchymal stem/progenitor cell-specific antigens for a predetermined period of incubation time;
isolating only cells bound to their corresponding antibodies using a cell sorter; cultivating the isolated cells; and
culturing in cell differentiation medium for a predetermined period of incubation time the mesenchymal stem/progenitor cells for the differentiation into chondrocytes.

2. The method of claim 1, wherein the mesenchymal stem/progenitor cells are isolated from the mononuclear cells by using antibody-binding specificity for an antibody(s) which is (are) one or more selected from antibodies against CD105, SH3 and SH4 antigens.

3. The method of claim 2, wherein the mesenchymal stem/progenitor cells are **characterized by** their ability to minimize the rejection response in case of tissue or organ transplantation.

4. The method of any one of claims 1 to 3, wherein the umbilical cord blood-derived mesenchymal stem/progenitor cells show a positive response to antibodies for CD29, CD49e, CD44, CD54, CD13, CD90, SH2, SH3 and SH4 antigens, and show a negative response to antibodies for CD45, CD34, CD14, HLA-DR, CD31, CD51/61, CD49d, CD106 and CD64 antigens.

5. The method of claim 4, wherein the mesenchymal stem/progenitor cells are **characterized by** their ability to minimize the side effect caused by production of undesired blood vessels or the differentiation into osteoclasts during the chondrogenesis in case of tissue or organ transplantation.

6. The method of claim 1, wherein the cell differentiation medium consists of 10 ng/ml of TGF-βIII, 6.25 µg/ml of bovine insulin, 6.25 µg/ml of transferrin, 5.35 µg/ml of selenous acid, 1.25 µg/ml of linoleic acid, 100 µg/ml of bovine serum albumin (BSA), 100 mM of sodium pyruvate, 100 nM of dexamethasone, 50 µg/ml of ascorbic acid 2-phosphate and 40 µg/ml of proline and a conventional cell culture medium.

7. The method of claim 6, wherein the conventional cell culture medium is selected from DMEM, α-MEN, McCoys 5A medium, Eagle's basal medium, CMRL medium, Glasgow minimal essential medium, Ham's F-12 medium, Iscove's modified Dulbecco's medium, Liebovitz' L-15 medium, RPMI 1640 medium.

8. The method of claims 6 or 7, wherein the cell differentiation medium further contains one or more assistants selected from a growth factor, horse or human serum, antibiotics and antifungal agents, including penicillin G, streptomycin sulfate, amphotericin B, gentamycin and nystatin.

## Patentansprüche

1. Verfahren zur Isolierung, Kultivierung und Chondrogenese von mesenchymalen Stamm-/Vorläuferzellen aus Nabelschnurblut, das die Schritte umfasst: Auftragen von Nabelschnurblut auf Ficoll-Hypaque Lösung; Zentrifugation des Nabelschnurbluts auf der Ficoll-Hypaque Lösung, um mononukleäre Zellen zu erhalten;
zur Reaktion bringen von Zellen, die durch Monolayerkultur der mononukleären Zellen erhalten wurden, mit Antikörpern gegen mesenchymale stamm-/vorläuferzellenspezifische Antigene für eine vorbestimmte Inkubationszeitspanne;
Isolierung nur der Zellen, die an ihre entsprechenden Antikörper gebunden sind, unter Verwendung eines Zellsorter;
Kultivierung der isolierten Zellen; und
Kultivierung der mesenchymalen Stamm-/Vorläuferzellen in Zelldifferenzierungsmedium, für eine vorbestimmte Inkubationszeitspanne, zur Ausdifferenzierung in Chondrozyten.

2. Das Verfahren nach Anspruch 1, wobei die mesenchymalen Stamm-/Vorläuferzellen von den mononukleären Zellen unter Ausnutzung von spezifischer Antikörperbindung an einen oder mehrere Antikörper, der/die ausgesucht ist/sind aus Antikörpern gegen CD 105, SH3 und SH4 Antigene.

3. Das Verfahren nach Anspruch 2, wobei die mesenchymalen Stamm-/Vorläuferzellen durch ihre Fähigkeit **gekennzeichnet** sind, die Abstoßungsreaktion im Fall von Gewebe- oder Organtransplantation zu minimieren.

4. Das Verfahren nach einem der Ansprüche 1 bis 3, wobei die aus Nabelschnurblut erhaltenen mesenchymalen Stamm-/Vorläuferzellen eine positive Antwort auf Antikörper für CD29, CD49e, CD44, CD54, CD13, CD90, SH2, SH3 und SH4 Antigene zeigen, und eine negative Antwort auf Antikörper für CD45, CD34, CD14, HLA-DR, CD31, CD51/61, CD49d, CD106 und CD64 Antigene zeigen.

5. Das Verfahren nach Anspruch 4, wobei die mesenchymalen Stamm-/Vorläuferzellen durch ihre Fähigkeit charakterisiert sind, die Nebenwirkung zu minimieren, die durch die Produktion von unerwünschten Blutgefäßen oder die Ausdifferenzierung in Osteoklasten während der Chondrogenese im Fall von Gewebe- oder Organtransplantation verursacht werden.

6. Das Verfahren nach Anspruch 1, wobei das Zelldifferenzierungsmedium aus 10 ng/ml TGF-βIII, 6,25 µg/ml Rinderinsulin, 6,25 µg/ml Transferrin, 5,35 µg/ml Seleniger Säure, 1,25 µg/ml Linolsäure, 100 µg/ml Rinderserumalbumin (BSA), 100 mM Natriumpyruvat, 100 nM Dexamethason, 50 µg/ml Ascorbinsäure-2-Phosphat und 40 µg/ml Prolin und einem herkömmlichen Zellkulturmedium besteht.

7. Das Verfahren nach Anspruch 6, wobei das herkömmliche Zellkulturmedium ausgewählt ist aus DMEM, α-MEN, McCoys 5A Medium, Eagles Basal Medium, CMRL Medium, Glasgow Minimalmedium, Hams F-12 Medium, Iscove's modified Dulbecco's Medium, Liebovitz L-15 Medium, RPMI 1640 Medium.

8. Das Verfahren nach Ansprüchen 6 oder 7, wobei das Zelldifferenzierungsmedium weiter ein oder mehrere Hilfsstoffe enthält, ausgewählt aus einem Wachstumsfaktor, Pferde- oder Humanserum, Antibiotika und antimykotischen Wirkstoffen, einschließlich Penicillin G, Streptomycinsulfat, Amphotericin B, Gentamycin und Nystatin.

## Revendications

1. Procédé pour l'isolement, la culture et la chondrogenèse de cellules souches/progénitrices mésenchymateuses provenant de sang de cordon ombilical, qui comprend les étapes consistant à :
verser une couche de sang de cordon ombilical sur une solution de Ficoll-Hypaque ;
centrifuger le sang de cordon ombilical sur la solution de Ficoll-Hypaque pour obtenir des cellules mononucléées ;
faire réagir les cellules obtenue par culture en couche monocellulaire des cellules mononucléées avec des anticorps contre des antigènes spécifiques des cellules souches/progénitrices mésenchymateuses pendant une période de temps d'incubation prédéterminée ;
isoler seulement les cellules liées à leurs anticorps correspondants en utilisant un appareil de tri cellulaire ;
cultiver les cellules isolées ; et
cultiver, dans un milieu de différentiation cellulaire, pendant une période de temps d'incubation prédéterminée, les cellules souches/progénitrices mésenchymateuses pour la différentiation en chondrocytes.

2. Procédé suivant la revendication 1, dans lequel les cellules souches/progénitrices mésenchymateuses sont isolées des cellules mononucléées en utilisant la spécificité de liaison d'anticorps pour un ou plusieurs anticorps qui consistent en un ou plusieurs anticorps choisis parmi des anticorps contre les antigènes CD105, SH3 et SH4.

3. Procédé suivant la revendication 2, dans lequel les cellules souches/progénitrices mésenchymateuses sont **caractérisées par** leur aptitude à réduire au minimum la réponse de rejet dans le cas de la transplantation d'un tissu ou organe.

4. Procédé suivant l'une quelconque des revendications 1 à 3, dans lequel les cellules souches/progénitrices mésenchymateuses dérivées de sang de cordon ombilical présentent une réponse positive aux anticorps pour les antigènes CD29, CD49e, CD44, CD54, CD13, CD90, SH2, SH3 et SH4, et présentent une réponse négative aux anticorps pour les antigènes CD45, CD34, CD14, HLA-DR, CD31, CD51/61, CD49d, CD106 et CD64.

5. Procédé suivant la revendication 4, dans lequel les cellules souches/progénitrices mésenchymateuses sont **caractérisées par** leur aptitude à réduire au minimum l'effet secondaire provoqué par la production de vaisseaux sanguins indésirables ou la différentiation en ostéoclastes au cours de la chondrogenèse dans le cas d'une transplantation de tissu ou d'organe.

6. Procédé suivant la revendication 1, dans lequel le milieu de différentiation cellulaire consiste en 10 ng/ml de TGF-βIII, 6,25 µg/ml d'insuline bovine, 6,25 µg/ml de transferrine, 5,35 µg/ml d'acide sélénieux, 1,25 µg/ml d'acide linoléique, 100 µg/ml de sérumalbumine bovine (SAB), 100 mM de pyruvate de sodium, 100 nM de dexaméthasone, 50 µg/ml de 2-phosphate d'acide ascorbique et 40 µg/ml de proline et un milieu classique de culture cellulaire.

7. Procédé suivant la revendication 6, dans lequel le milieu classique de culture cellulaire est choisi entre le milieu DMEM, le milieu α-MEN, le milieu 5A de McCoy, le milieu basal de Eagle, le milieu CMRL, le milieu essentiel minimal de Glasgow, le milieu F-12 de Ham, le milieu de Dulbecco modifié par Iscove, le milieu L-15 de Liebovitz et le milieu RPMI 1640.

8. Procédé suivant la revendication 6 ou 7, dans lequel le milieu de différentiation cellulaire contient en outre un ou plusieurs auxiliaires choisis entre un facteur de croissance, du sérum de cheval ou humain, des antibiotiques et des agents antifongiques, notamment la pénicilline G, le sulfate de streptomycine, l'amphotéricine B, la gentamycine et la nystatine.


### Sequence Listing

<110> Medipost
<120> Isolation and culture-expansion methods of mesenchymal stem/progenitor cells from umbilical cord blood, and differentiation method of umbilical cord blood-derived mesenchymal stem/progenitor cells into various mesenchymal tissues
<130> 03PP018
<150> KR 10-2002-0008639
   <151> 2002-02-19
<150> KR 10-2003-0010269
   <151> 2003-02-19
<160> 16
<170> KopatentIn 1.71
<210> 1
   <211> 20
   <212> DNA
   <213> Artificial Sequence
<220>
   <223> forward primer for amplifying GAPDH gene
<400> 1
   accacagtcc atgccatcac 20
<210> 2
   <211> 20
   <212> DNA
   <213> Artificial Sequence
<220>
   <223> reverse primer for amplifying GAPDH gene
<400> 2
   tccaccaccc tgttgctgta 20
<210> 3
   <211> 21
   <212> DNA
   <213> Artificial Sequence
<220>
   <223> forward primer for amplifying type 1 collagen gene
<400> 3
   ccccctcccc agccacaaag a 21
<210> 4
   <211> 21
   <212> DNA
   <213> Artificial Sequence
<220>
   <223> reverse primer for amplifying type 1 collagen gene
<400> 4
   tcttggtcgg tggtggactc t 21
<210> 5
   <211> 20
   <212> DNA
   <213> Artificial Sequence
<220>
   <223> forward primer for amplifying type 2 collagen gene
<400> 5
   tttcccaggt caagatggtc 20
<210> 6
   <211> 20
   <212> DNA
   <213> Artificial Sequence
<220>
   <223> reverse primer for amplifying type 2 collagen gene
<400> 6
   cttcaccacc tgtctcacca 20
<210> 7
   <211> 21
   <212> DNA
   <213> Artificial Sequence
<220>
   <223> forward primer for amplifying type 10 collagen gene
<400> 7
   ccctttttgc tgctagtatc c 21
<210> 8
   <211> 24
   <212> DNA
   <213> Artificial Sequence
<220>
   <223> reverse primer for amplifying type 10 collagen gene
<400> 8
   ctgttgtcca ggttttcctg gcac 24
<210> 9
   <211> 23
   <212> DNA
   <213> Artificial Sequence
<220>
   <223> forward primer for amplifying aggrecan gene
<400> 9
   tgaggagggc tggaacaagt acc 23
<210> 10
   <211> 23
   <212> DNA
   <213> Artificial Sequence
<220>
   <223> reverse primer for amplifying aggrecan gene
<400> 10
   ggaggtggta attgcaggga aca 23
<210> 11
   <211> 16
   <212> DNA
   <213> Artificial Sequence
<220>
   <223> forward primer for amplifying osteocalcin gene
<400> 11
   catgacagcc ctcaca 16
<210> 12
   <211> 17
   <212> DNA
   <213> Artificial Sequence
<220>
   <223> reverse primer for amplifying osteocalcin gene
<400> 12
   agagcgacac cctagac 17
<210> 13
   <211> 20
   <212> DNA
   <213> Artificial Sequence
<220>
   <223> forward primer for amplifying osteopontin gene
<400> 13
   ccaagtaagt ccaacgaaag 20
<210> 14
   <211> 19
   <212> DNA
   <213> Artificial Sequence
<220>
   <223> reverse primer for amplifying osteopontin gene
<400> 14
   ggtgatgtcc tcgtctgta 19
<210> 15
   <211> 24
   <212> DNA
   <213> Artificial Sequence
<220>
   <223> forward primer for amplifying alkaline phosphatase gene
<400> 15
   tggagcttca gagactcaac acca 24
<210> 16
   <211> 24
   <212> DNA
   <213> Artificial Sequence
<220>
   <223> reverse primer for amplifying alkaline phosphatase gene
<400> 16
   atctcgttgt ctgagtacca gtcc 24